(19) Europäisches Patentamt
European Patent Office
Office européen des brevets

(11) **EP 3 972 735 B1**

(12) **EUROPÄISCHE PATENTSCHRIFT**

(45) Veröffentlichungstag und Bekanntmachung des Hinweises auf die Patenterteilung:
**16.08.2023   Patentblatt 2023/33**

(21) Anmeldenummer: **20726142.1**

(22) Anmeldetag: **20.05.2020**

(51) Internationale Patentklassifikation (IPC):
**B01J 35/04** (2006.01)   **B01J 35/10** (2006.01)
**B01J 35/02** (2006.01)

(52) Gemeinsame Patentklassifikation (CPC):
(C-Sets verfügbar)
**B01J 35/04; B01J 35/023; B01J 35/10; C07C 1/12**

(Forts.)

(86) Internationale Anmeldenummer:
**PCT/EP2020/064040**

(87) Internationale Veröffentlichungsnummer:
**WO 2020/234337 (26.11.2020 Gazette 2020/48)**

(54) **KATALYSATOR FÜR EXOTHERME REAKTIONEN**

CATALYSTS FOR EXOTHERMIC REACTIONS

CATALYSEUR POUR RÉACTIONS EXOTHERMIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.05.2019   EP 19175573**

(43) Veröffentlichungstag der Anmeldung:
**30.03.2022   Patentblatt 2022/13**

(73) Patentinhaber: **Max-Planck-Gesellschaft zur Förderung der Wissenschaften e.V.
80539 München (DE)**

(72) Erfinder:
• **ZIMMERMANN, Ronny Tobias
39106 Magdeburg (DE)**
• **BREMER, Jens
39108 Magdeburg (DE)**
• **SUNDMACHER, Kai
38350 Helmstedt (DE)**

(74) Vertreter: **Maiwald GmbH
Elisenhof
Elisenstraße 3
80335 München (DE)**

(56) Entgegenhaltungen:
WO-A1-2017/161953      DE-A1- 2 813 329
US-A1- 2012 302 811      US-B1- 6 211 255

(52) Gemeinsame Patentklassifikation (CPC): (Forts.)

C-Sets
**C07C 1/12, C07C 9/04**

**Beschreibung**

Die vorliegende Erfindung betrifft Katalysatorformkörper und deren Verwendung für exotherme Gasphasenreaktionen.

Für die Katalyse industrieller Prozesse werden üblicherweise makroskopische Formkörper mit Abmessungen im Millimeter- oder Zentimeterbereich verwendet. In Festbettreaktoren können pulverförmige Katalysatoren zu einem starken Druckabfall führen. Eine Übersicht gebräuchlicher Katalysatorformkörper für die heterogene Katalyse findet sich beispielsweise in "Industrial Catalysis - A Practical Approach", Kapitel 6: "Catalyst Shapes and Production of Heterogeneous Catalysts", Autor: Jens Hagen, Wiley-VCH Verlag, 2006, sowie "Heterogeneous Catalysis and Solid Catalysts", H. Knözinger et al., in Ullmann's Encyclopedia of Industrial Chemistry, Wiley-VCH Verlag, 2009.

Je nach Verteilung des katalytisch aktiven Materials in dem Katalysatorformkörper wird beispielsweise zwischen einer homogenen Struktur, einer "Egg-Yolk"-Struktur und einer "Egg-Shell"-Struktur unterschieden. In der homogenen Struktur ist das katalytisch aktive Material gleichmäßig in dem Formkörper verteilt. In der Egg-Yolk-Struktur liegt das katalytisch aktive Material im Kern des Förmkörpers vor und ist von einer katalytisch inaktiven Hülle umgeben. In der Egg-Shell-Struktur weist der Katalysatorformkörper einen katalytisch inaktiven Kern auf, der von einer katalytisch aktiven Hülle umgeben ist.

Bei vielen industriell relevanten chemischen Prozessen handelt es sich um eine exotherme Gasphasen-Reaktion, beispielsweise eine Hydrierung (z.B. eine Methanisierung), eine Oxidation, eine Acetylierung, eine Aminierung oder eine Nitrilierung. Prinzipiell wird dabei eine hohe Raum-Zeit-Ausbeute angestrebt. Andererseits besteht bei exothermen Reaktionen die Gefahr, dass sich im Reaktor "Hot-Spots", d.h. lokale Bereiche mit sehr hoher Temperaturbelastung, ausbilden, die zu einer Schädigung des Katalysators führen können.

Eine Übersicht zur Methanisierung als einer der industriell relevanten exothermen Gasphasenreaktionen findet sich bei S. Rönsch et al., Fuel, 166, 2016, S. 276-296. Bei der Methanisierung werden Kohlenstoffoxide (CO und/oder $CO_2$) mit Wasserstoff in einer stark exothermen Reaktion zu Methan umgesetzt. Die folgende Reaktionsgleichung gibt die Umsetzung von $CO_2$ mit Wasserstoff wieder:

$$CO_2 + 4\,H_2 \rightarrow CH_4 + 2\,H_2O$$

Aufgrund thermodynamischer Einschränkungen, Katalysatordeaktivierung und Sicherheitsaspekten wurden verschiedene Reaktorkonzepte entwickelt, um die Wärme, die durch heterogen katalysierte, exotherme Reaktionen freigesetzt wird, effizient abführen zu können. Ein weit verbreitetes Konzept sind Festbett-Rohrreaktoren. Diese bestehen häufig aus wandgekühlten Rohren, die mit katalytisch aktiven Formkörpern gefüllt sind. In der Regel werden viele dieser Rohre zu einem Rohrbündelreaktor zusammengefasst.

Bei erhöhten Wärmefreisetzungsraten ist es trotz Wandkühlung nicht möglich, diese Reaktoren, selbst bei kleinen Rohrdurchmessern, ohne einen ausgeprägten Hot-Spot zu betreiben. Jedoch sollten ausgeprägte Hot-Spots aus den vorher genannten Gründen und wegen zusätzlicher Kosten für hochtemperaturbeständige Materialien vermieden werden.

Ein bekannter Lösungsansatz zur Vermeidung von Hot-Spots ist die Injektion von Produkt- oder Inertgas in den Reaktorfeed. Durch die Verdünnung wird die freiwerdende Reaktionswärme reduziert und daher der Temperaturhub im Reaktor verringert. Nachteilig bei dieser Vorgehensweise ist die reduzierte Raum-Zeit-Ausbeute. Weiterhin erfordert die Verdünnung mit Inertgas eine nachfolgende Aufbereitung des Produktstroms, wodurch die Prozesskosten erhöht werden. Im Gegenzug erfordert die Verdünnung mit Produktgas einen zusätzlichen Kompressor, um den Reaktordruckverlust zu kompensieren.

Aus wirtschaftlicher Sicht ist es daher vorteilhaft, die Verdünnung der Edukte so klein wie möglich zu halten. Hierfür wurden verschiedene Reaktorkonzepte untersucht. Beispiele sind die Verwendung mehrerer Kühlzonen, mehrere adiabate Festbett-Reaktoren in Serie, strukturierte Katalysatorpackungen, Katalysatorverdünnung, Membranreaktoren oder verteilte Eduktgasinjektion. Diese Konzepte erfordern häufig einen erhöhten Fertigungsaufwand.

Neben der konventionellen stationären Betriebsweise von Reaktoren ist im Rahmen der Energiewende die dynamische Betriebsweise von Reaktoren in den Fokus der Forschung gerückt. Elektrische Energie, die zum Beispiel aus Sonnen- und Windenergie gewonnen wird, soll durch "*Power-to-X*"-Prozesse in chemische Energieträger umgewandelt werden. Zur Vermeidung von großen Zwischenspeichern für Intermediate ist ein dynamischer Betrieb von Festbettreaktoren, je nach Verfügbarkeit überschüssiger Energie, bevorzugt. Jedoch ergeben sich hierbei zusätzliche Herausforderungen beim Betrieb der Festbett-Reaktoren, beispielsweise durch inverses Verhalten oder Hysterese-Phänomene.

S. Hwang et al., Chem. Eng. Comm., 196, 2009, S. 616-642, beschreiben einen Katalysator-Formkörper für eine wasserstoffreiche Methanisierung. In Simulationen werden verschiedene Katalysator-Formkörper mit inhomogener Verteilung des katalytisch aktiven Materials beschrieben. Der Katalysator-Formkörper weist eine homogene Porenstruktur auf.

L. Kershenbaum et al., Chemical Engineering Science, 56, 2001, S. 651-658, beschreiben ein System, das eine keramische Oxid-Brennstoffzelle und einen Reaktor für eine Dampfreformierung enthält. Bei der Dampfreformierung wird in einer stark endothermen Reaktion Wasser mit Methan zu $CO/CO_2$ und Wasserstoff umgesetzt. Der Wasserstoff kann der Brennstoffzelle zugeführt werden. Für die endotherme Dampfreformierung wird ein Katalysator-Formkörper beschrieben, der eine äußere katalytisch inaktive, poröse Beschichtung aufweist, die den katalytisch aktiven Bereich im Inneren des Formkörpers umschließt. Genauere Angaben über die Porositäten und Porendurchmesser in diesen unterschiedlichen Bereichen des Katalysator-Formkörpers werden nicht gemacht.

WO 2017/161953 A1 beschreibt einen katalysatorhaltigen Formkörper, der eine wabenförmige Keramik, eine auf der Keramik aufgebrachte Beschichtung (beispielsweise $\gamma$-$Al_2O_3$) und eine auf der Beschichtung aufgebrachte katalytisch aktive Komponente enthält. Um einen bifunktionalen Katalysator zu erhalten, wird die wabenförmige Keramik in ein unteres Segment und ein oberes Segment unterteilt und auf diesen Segmenten unterschiedliche katalytisch aktive Komponente aufgebracht.

DE 28 13 329 A1 beschreibt einen katalysatorhaltigen Formkörper, der ein gewelltes metallisches Trägermaterial und eine auf dem gewellten Metall aufgebrachte poröse Beschichtung (beispielsweise $\gamma$-$Al_2O_3$), in der die katalytisch aktive Komponente vorliegt, enthält.

US 2012/302811 A1 beschreibt einen Formkörper, der ein Metallsubstrat, eine auf dem Metallsubstrat vorliegende dichte Aluminiumoxidschicht und eine auf der Aluminiumoxidschicht aufgebrachte katalytisch aktive Komponente enthält.

US 6 211 255 B1 beschreibt ein Fischer-Tropsch-Syntheseverfahren unter Verwendung eines katalysatorhaltigen Formkörpers. Beispielsweise enthält der Formkörper eine Keramik und eine poröse Beschichtung, in der das katalytisch aktive Material vorliegt.

Eine Aufgabe der vorliegenden Erfindung ist die effiziente Katalyse exothermer Reaktionen (wie z.B. der Methanisierung), wobei ein möglichst guter Kompromiss zwischen hoher Raum-Zeit-Ausbeute und ausreichend niedriger Katalysatortemperatur (d.h. Vermeidung von "Hot Spots", die den Katalysator schädigen könnten) erzielt werden soll. Der Katalysator sollte für industrielle chemische Prozesse geeignet sein und ein effizientes Betreiben des Reaktors auch unter dynamischen Bedingungen und verschiedenen Lastzuständen ermöglichen.

Gelöst wird die Aufgabe durch einen Formkörper, der ein katalytisch aktives Material enthält und der einen katalytisch aktiven Bereich sowie eine poröse Beschichtung, die auf dem katalytisch aktiven Bereich vorliegt, umfasst, wobei

- mindestens 75 Gew% des katalytisch aktiven Materials des Formkörpers in dem katalytisch aktiven Bereich vorliegen, und
- die poröse Beschichtung und der katalytisch aktive Bereich der folgenden Bedingung genügt:

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1{,}0.$$

wobei

$\Theta_{PB}$ die Porosität der porösen Beschichtung ist;
$d_{PB}$ der mittlere Porendurchmesser, in nm, der porösen Beschichtung ist;
$\Theta_{KB}$ die Porosität des katalytisch aktiven Bereichs ist;
$d_{KB}$ der mittlere Porendurchmesser, in nm, des katalytisch aktiven Bereichs ist.

Im Rahmen der vorliegenden Erfindung wurde erkannt, dass bei einer exothermen chemischen Reaktion die Anwesenheit einer porösen und im Wesentlichen katalytisch inaktiven Beschichtung ("poröse Schale"), die den katalytisch aktiven Kern des Formkörpers umgibt, einen vorteilhaften Einfluss auf die Katalysatortemperatur hat und gleichzeitig die Raum-Zeit-Ausbeute auf einem hohen oder zumindest akzeptablen Niveau hält. Eine weitere Absenkung der Katalysatortemperatur bei weiterhin ausreichend hohen Raum-Zeit-Ausbeuten wird realisiert, wenn die Porositäten und mittleren Porendurchmesser des katalytisch aktiven Kerns und der im Wesentlichen katalytisch inaktiven Schale so aufeinander abgestimmt werden, dass eine chemische Verbindung in der Schale einen geringeren effektiven Diffusionskoeffizienten aufweist als im Kern des Formkörpers. Der effektive Diffusionskoeffizient ist ein Maß für die Beweglichkeit einer Verbindung in einem porösen Material und kann durch den Porendurchmesser und/oder die Porosität eingestellt werden.

Es hat sich im Rahmen der vorliegenden Erfindung überraschend gezeigt, dass bei exothermen chemischen Reaktionen ein verbesserter Kompromiss zwischen hoher Raum-Zeit-Ausbeute und ausreichend niedriger Katalysatortemperatur (d.h. Vermeidung von "Hot Spots") erzielt wird, wenn die Porositäten und mittleren Porendurchmesser des katalytisch aktiven Kerns und der ihn umgebenden porösen Beschichtung, die im Wesentlich kein katalytisch aktives Material enthält, so aufeinander abgestimmt sind, dass sie der oben genannten Beziehung genügen, d.h.

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1{,}0.$$

Als katalytisch aktiver Bereich fungiert beispielsweise ein katalytisch aktiver Ausgangsformkörper, der kommerziell erhältlich ist oder über Standardverfahren, die dem Fachmann bekannt sind, hergestellt wird. In dem katalytisch aktiven Ausgangsformkörper kann das katalytisch aktive Material beispielsweise homogen verteilt sein. Alternativ ist es auch möglich, dass das katalytisch aktive Material in dem Ausgangsformkörper inhomogen verteilt ist, beispielsweise in Form einer Egg-Shell-Struktur (d.h. einer katalytisch aktiven Schale, die ein katalytisch inaktives Material umschließt). Auf diesen katalytisch aktiven Ausgangsformkörper wird eine poröse Beschichtung, die kein oder nur sehr wenig katalytisch aktives Material enthält, aufgebracht. Unter Berücksichtigung der Porosität und des mittleren Porendurchmessers des katalytisch aktiven Ausgangsformkörpers werden Porosität und mittlerer Porendurchmesser der auf dem Ausgangsformkörper aufgebrachten porösen Beschichtung so gewählt, dass die oben genannte Beziehung erfüllt ist.

Der katalytisch aktive Bereich umfasst beispielsweise ein poröses Trägermaterial, auf dem das katalytisch aktive Material dispergiert ist. In Abhängigkeit von der durchzuführenden exothermen chemischen Reaktion ist dem Fachmann bekannt, welche katalytisch aktiven Materialien jeweils geeignet sind. Wie oben bereits erwähnt, sind katalytisch aktive Ausgangsformkörper, die ein poröses Trägermaterial und ein auf diesem Trägermaterial dispergiertes katalytisch aktives Material umfassen, kommerziell und/oder über bekannte Herstellungsverfahren erhältlich. Eine Übersicht hinsichtlich der Herstellung katalytisch aktiver Formkörper findet sich z.B. in der Publikation von F. Schüth, M. Hesse, "Catalyst Forming", in Handbook of Heterogeneous Catalysis, S. 676-699, Wiley-VCH, Weinheim (2008), Ed.: G. Ertl, H. Knözinger, F. Schüth, J. Weitkamp.

Beispielhafte poröse Trägermaterialien, die in dem katalytisch aktiven Bereich vorliegen können, sind Oxide (wie z.B. $Al_2O_3$ oder $SiO_2$), Nitride, Carbide und/oder elementarer Kohlenstoff.

Das katalytisch aktive Material wird in Abhängigkeit von der zu katalysierenden exothermen Reaktion ausgewählt. Beispielhaft können in diesem Zusammenhang Metalle (z.B. Übergangsmetalle) und Übergangsmetalloxide genannt werden.

Der katalytisch aktive Bereich des erfindungsgemäßen Katalysator-Formkörpers weist beispielsweise eine Porosität $\Theta_{KB}$ im Bereich von 0,2 bis 0,8, bevorzugter 0,3 bis 0,7 auf.

Der mittlere Porendurchmesser $d_{KB}$ des katalytisch aktiven Bereichs kann über einen relativ breiten Bereich variieren. Der katalytisch aktive Bereich kann Mikroporen, Mesoporen und/oder Makroporen enthalten. Gemäß IUPAC-Definition weisen Mikroporen einen Durchmesser < 2nm, Mesoporen einen Durchmesser von 2 bis 50 nm und Makroporen einen Durchmesser > 50 nm auf. Beispielsweise weist der katalytisch aktive Bereich einen mittleren Porendurchmesser $d_{KB}$ von 1 nm bis 2000 nm, bevorzugter 2 nm bis 1200 nm oder 2 nm bis 500 nm auf.

In Kenntnis der Porosität und des mittleren Porendurchmessers des katalytisch aktiven Ausgangsformkörpers können die Porosität und der mittlere Porendurchmesser der auf dem Ausgangsformkörper aufzubringenden porösen Beschichtung gewählt werden, so dass in dem Formkörper der vorliegenden Erfindung die folgende Bedingung erfüllt ist:

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1{,}0$$

Bevorzugt gilt:

$$[\Theta_{PB}^{1.5} \times d_{PB} \times (d_{KB} + 150\ nm)] / [\Theta_{KB}^{1.5} \times d_{KB} \times (d_{PB} + 150\ nm)] \leq 0{,}75$$

oder

$$[\Theta_{PB}^{1.5} \times d_{PB} \times (d_{KB} + 150\ nm)] / [\Theta_{KB}^{1.5} \times d_{KB} \times (d_{PB} + 150\ nm)] \leq 0{,}5$$

Noch bevorzugter gilt:

$$0{,}01 < [\Theta_{PB}^{1.5} \times d_{PB} \times (d_{KB} + 150\ nm)] / [\Theta_{KB}^{1.5} \times d_{KB} \times (d_{PB} + 150\ nm)] < 1{,}0$$

oder

$$0.01 < [\Theta_{PB}^{1.5} \times d_{PB} \times (d_{KB} + 150 \text{ nm})] / [\Theta_{KB}^{1.5} \times d_{KB} \times (d_{PB} + 150 \text{ nm})] \le 0.75$$

oder

$$0.01 < [\Theta_{PB}^{1.5} \times d_{PB} \times (d_{KB} + 150 \text{ nm})] / [\Theta_{KB}^{1.5} \times d_{KB} \times (d_{PB} + 150 \text{ nm})] \le 0.5$$

Geeignete Verfahren zur Herstellung poröser Beschichtungen sind dem Fachmann bekannt. Auch ist dem Fachmann bekannt, durch welche Maßnahmen die Porosität $\Theta_{PB}$ und der mittlere Porendurchmesser $d_{PB}$ einer porösen Beschichtung beeinflusst werden können.

Die poröse Beschichtung kann beispielsweise über ein Sol-Gel-Verfahren hergestellt werden. Die Herstellung poröser Materialien über Sol-Gel-Verfahren wird beispielsweise beschrieben in:

- M.V. Landau, "Sol-Gel Process", S. 119-160, in "Handbook of heterogeneous catalysis", 2008, Vol. 1, Wiley-VCH Verlag,
- C.J. Brinker et al., Journal of Membrane Science, 94, 1994, S. 85-102.
- F. Schüth, "General Principles for the Synthesis and Modification of Porous Materials", S. 622-629, in "Handbook of Porous Solids", Ed.: F. Schüth, S.W. Sing, J. Weitkamp, Vol. 1, Wiley-VCH, 2002.

Weiterhin ist es möglich, die poröse Beschichtung über eine Sprühbeschichtung herzustellen. Die Sprühbeschichtung kann beispielsweise in einer Wirbelschichtkammer erfolgen. Solche Beschichtungsverfahren sind dem Fachmann bekannt. Beispielsweise wird die den katalytisch aktiven Bereich bildende Komponente (z.B. ein poröses Trägermaterial, auf dem das katalytisch aktive Material dispergiert ist) in einer Wirbelschichtkammer fluidisiert und eine Suspension eines partikelförmigen Materials, das die poröse Beschichtung bildet, wird in die Wirbelschichtkammer eingedüst. Durch die Düse wird die Suspension fein zerstäubt. Das partikelförmige Material der eingedüsten Suspension lagert sich auf dem katalytisch aktiven Trägermaterial ab und bildet die poröse Beschichtung. Die Dicke der aufgebrachten porösen Beschichtung kann beispielsweise durch die Dauer des Beschichtungsprozesses in der Wirbelschichtkammer und die Eindüsungsrate der Suspension variiert werden. Nach erfolgter Beschichtung in der Wirbelschichtkammer können die Formkörper noch einer thermischen Behandlung unterzogen werden. Über diese thermische Behandlung können Porendurchmesser und/oder Porosität der porösen Beschichtung beeinflusst werden. Der Porendurchmesser und/oder die Porosität können auch durch die Partikelgröße des partikelförmigen Materials der eingedüsten Suspension beeinflusst werden. Durch Verwendung eines unporösen Füllmaterials kann gegebenenfalls die Porosität verringert werden.

Für die poröse Beschichtung können Materialien verwendet werden, wie sie auch für das Trägermaterial im katalytisch aktiven Bereich verwendet werden. Beispielsweise enthält die poröse Beschichtung ein Oxid (wie z.B. $Al_2O_3$, $SiO_2$, ein Erdalkalimetalloxid wie Magnesiumoxid, ein Seltenerdmetalloxid wie Ceroxid), ein Nitrid, ein Carbid und/oder elementaren Kohlenstoff.

Die poröse Beschichtung stellt bevorzugt die äußerste Beschichtung des Formkörpers dar, d.h. auf der porösen Beschichtung liegt bevorzugt keine weitere Beschichtung vor. Die poröse Beschichtung liegt bevorzugt direkt auf dem katalytisch aktiven Bereich vor. Weiterhin ist bevorzugt, dass zumindest 90%, bevorzugter zumindest 95% der Fläche des katalytisch aktiven Bereichs von der porösen Beschichtung umschlossen sind. In einer bevorzugten Ausführungsform ist der katalytisch aktive Bereich vollständig von der porösen Beschichtung umschlossen.

Da der Formkörper als Katalysator für industrielle chemische Prozesse in entsprechenden Reaktoren (z.B. einem Festbettreaktor) geeignet sein soll, liegen seine Abmessungen bevorzugt im Millimeter- oder Zentimeterbereich. Der Formkörper der vorliegenden Erfindung weist bevorzugt einen volumenäquivalenten Kugeldurchmesser im Bereich von 1,0 mm bis 50 mm, bevorzugter 1,0 bis 20 mm auf. Wie allgemein bekannt ist, gibt der volumenäquivalente Kugeldurchmesser den Durchmesser einer Kugel an, die das gleiche Volumen wie der betrachtete Körper aufweist.

Die poröse Beschichtung des Formkörpers weist beispielsweise eine Schichtdicke im Bereich von 10 μm bis 2000 μm, bevorzugter von 50 μm bis 1000 μm auf. Wird eine zu dünne poröse Beschichtung verwendet, kann sich dies nachteilig auf die Katalysatortemperatur auswirken, während sich eine zu dicke poröse Beschichtung nachteilig auf die Raum-Zeit-Ausbeute auswirken kann.

Prinzipiell können Porosität $\Theta_{PB}$ und mittlerer Porendurchmesser $d_{PB}$ der porösen Beschichtung über einen relativ breiten Bereich variiert werden, solange die oben genannte Bedingung, d.h.

$$[\Theta_{PB}^{1.5} \times d_{PB} \times (d_{KB} + 150 \text{ nm})] / [\Theta_{KB}^{1.5} \times d_{KB} \times (d_{PB} + 150 \text{ nm})] < 1.0$$

erfüllt ist. Um die Hot-Spot-Bildung am Katalysator-Formkörper aber noch effektiver zu verhindern, kann es im Rahmen der vorliegenden Erfindung bevorzugt sein, den mittleren Porendurchmesser der porösen Beschichtung so zu wählen, dass in den Poren überwiegend eine Knudsen-Diffusion stattfindet. Die poröse Beschichtung des Katalysator-Formkörpers weist beispielsweise einen mittleren Porendurchmesser $d_{PB} \leq 200$ nm, bevorzugter $\leq 150$ nm, noch bevorzugter $\leq 50$ nm auf. Beispielsweise liegt der mittlere Porendurchmesser $d_{PB}$ im Bereich von 2 nm bis 200 nm, bevorzugter 2 nm bis 150 nm, noch bevorzugter 2 nm bis 50 nm.

Bevorzugt liegen mindestens 85 Gew%, noch bevorzugter mindestens 95 Gew% oder sogar mindestens 99 Gew% des katalytisch aktiven Materials des Formkörpers in dem katalytisch aktiven Bereich vor. Im Rahmen der vorliegenden Erfindung ist es auch möglich, dass das gesamte katalytisch aktive Material in dem katalytisch aktiven Bereich vorliegt.

Der Formkörper kann eine Geometrie aufweisen, wie sie im Bereich der Katalyse allgemein üblich ist. Beispielsweise liegt der Formkörper in Form eines Pellets, einer Kugel, eines Rings, einer Tablette, eines Strangs, eines Zylinders, eines schwammförmigen Formkörpers oder eines wabenförmigen Formkörpers vor.

Weiterhin betrifft die vorliegende Erfindung die Herstellung des oben beschriebenen Formkörpers, wobei eine poröse Beschichtung auf einen Ausgangsformkörper, der ein katalytisch aktives Material enthält, aufgebracht wird.

Wie oben bereits erwähnt, kann die poröse Beschichtung beispielsweise über ein Sol-Gel-Verfahren aufgebracht werden.

Weiterhin betrifft die vorliegende Erfindung die Verwendung des oben beschriebenen Formkörpers als Katalysator für eine exotherme Gasphasenreaktion.

Die exotherme Reaktion weist beispielsweise eine Standardreaktionsenthalpie $\Delta H° \leq -10$ kJ/mol, bevorzugter $\leq -50$ kJ/mol, noch bevorzugter $\leq -100$ kJ/mol auf.

Beispielsweise ist die exotherme Gasphasenreaktion eine Hydrierung (z.B. eine Methanisierung), eine Oxidation, eine Acetylierung, eine Aminierung oder eine Nitrilierung ist.

Weiterhin betrifft die vorliegende Erfindung ein Verfahren zur exothermen Umsetzung gasförmiger Edukte in einem Reaktor, wobei die Edukte mit dem oben beschriebenen Formkörper in Kontakt gebracht werden.

Geeignete Reaktoren zur Durchführung exothermer Gasphasenreaktionen sind dem Fachmann bekannt. Beispielsweise ist der Reaktor ein Festbettreaktor, ein Wirbelschichtreaktor, ein Wandreaktor, ein Membranreaktor, ein Mikroreaktor, ein Wabenreaktor oder ein Plattenreaktor.

Wie oben bereits erwähnt, handelt es sich bei der exothermen Umsetzung gasförmiger Edukte beispielsweise um eine Hydrierung (z. B. eine Methanisierung), eine Oxidation, eine Acetylierung, eine Aminierung oder eine Nitrilierung. In Kenntnis der durchzuführenden Reaktion weiß der Fachmann, welche Edukte zu verwenden sind. Beispielsweise wird bei der Methanisierung $H_2$ mit $CO_2$ und/oder CO zu Methan umgesetzt.

Der Formkörper der vorliegenden Erfindung ermöglicht bei exothermen Reaktionen einen verbesserten Kompromiss zwischen einer möglichst hohen Raum-Zeit-Ausbeute und einer ausreichend niedrigen Katalysatortemperatur (d.h. Vermeidung von "Hot Spots"). Handelt es sich bei der exothermen Gasphasenreaktion beispielsweise um die Methanisierung, so ermöglicht das verbesserte Eigenschaftsprofil des Katalysators, dass die Edukte $CO_2$ und $H_2$ unverdünnt in einem molaren Verhältnis von $CO_2$ zu $H_2$ im Bereich von 1/3,5 bis 1/4,5 dem Reaktor zugeführt werden, ohne dass sich unerwünschte Hot-Spots am Katalysatormaterial bilden. Weiterhin ist es möglich, den Reaktor bei der Methanisierung nicht nur stationär, sondern auch mit Lastwechseln zu betreiben.

Messmethoden

Mittlerer Porendurchmesser und Porosität:
Die Bestimmung des mittleren Porendurchmessers (Medianwert) und der Porosität erfolgt mit der Quecksilberporosimetrie gemäß ISO 15901-1:2016. In der erweiterten Porosimetrie wird neben dem mittleren Porendurchmesser (als Medianwert) und des spezifischen Porenvolumens (d.h. Porenvolumen pro Masse der Probe) auch die Porosität bestimmt. Dabei wird mittels Pyknometrie (z.B. Quecksilber-Pyknometrie) zunächst die Rohdichte bestimmt. Die Porosität berechnet sich dann folgendermaßen:

$$\text{Porosität} = \text{spezifisches Porenvolumen} \times \text{Rohdichte}$$

Für die porosimetrische Bestimmung wird beispielsweise die poröse Beschichtung von dem katalytisch aktiven Bereich abgetrennt und beide Komponenten werden dann getrennt vermessen.

**Beispiele**

**Beispiel für die Herstellung eines erfindungsgemäßen Katalysator-Formkörpers**

Anhand dieses Beispiels wird die Herstellung eines erfindungsgemäßen Formkörpers eingehender erläutert.

Der katalytisch aktive Bereich des Formkörpers

Es wurden poröse $\gamma$-$Al_2O_3$-Kugeln mit einem Kugeldurchmesser von 2,5 mm (kommerziell erhältlich von Sasol) mit 9 Gew% Nickeloxid imprägniert. Die porösen $\gamma$-$Al_2O_3$-Kugeln fungieren als poröses Trägermaterial, auf dem das katalytisch aktive Material (Nickeloxid) dispergiert ist.

Über Quecksilberporosimetrie wurden der mittlere Porendurchmesser $d_{KB}$ und die Porosität $\Theta_{KB}$ der mit Nickeloxid beladenen $\gamma$-$Al_2O_3$-Kugeln bestimmt. Die Porosität $\Theta_{KB}$ betrug 0,687 und der mittlere Porendurchmesser $d_{KB}$ betrug 10,7 nm.

Aufbringen einer porösen Beschichtung auf dem katalytisch aktiven Bereich

In den erfindungsgemäßen Formkörpern genügen die poröse Beschichtung und der von dieser Beschichtung umgebene katalytisch aktive Bereich der folgenden Bedingung:

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1,0$$

wobei

$\Theta_{PB}$ die Porosität der porösen Beschichtung ist;
$d_{PB}$ der mittlere Porendurchmesser, in nm, der porösen Beschichtung ist;
$\Theta_{KB}$ die Porosität des katalytisch aktiven Bereichs ist;
$d_{KB}$ der mittlere Porendurchmesser, in nm, des katalytisch aktiven Bereichs ist.

Unter Berücksichtigung der Porosität $\Theta_{KB}$ und des mittleren Porendurchmessers $d_{KB}$ des oben beschriebenen katalytisch aktiven Bereichs ($\Theta_{KB}$ = 0,687; $d_{KB}$ = 10,7 nm) müssen die Porosität $\Theta_{PB}$ und der mittlere Porendurchmesser $d_{PB}$ der aufzubringenden porösen Beschichtung so gewählt werden, dass die erfindungsgemäße Bedingung erfüllt ist.

Wenn für die poröse Beschichtung beispielsweise eine eher niedrige Porosität von 40% gewählt wird, darf der mittlere Porendurchmesser der porösen Beschichtung einen Wert von 26,4 nm nicht überschreiten. Wenn alternativ für die poröse Beschichtung eine relativ hohe Porosität von 70% gewählt wird, darf der mittlere Porendurchmesser der porösen Beschichtung einen Wert von 10,4 nm nicht überschreiten.

In dem vorliegenden Beispiel wurde eine poröse $Al_2O_3$-Beschichtung über eine Sprühbeschichtung in einer Wirbelschichtkammer aufgebracht. Alternativ lassen sich Beschichtungen mit definiertem Porendurchmesser und definierter Porosität auch über ein dem Fachmann grundsätzlich bekanntes Sol-Gel-Verfahren aufbringen.

In der Wirbelschichtkammer (Durchmesser: 200 mm) wurden unter Verwendung von Luft als Fluidisierungsgas (Temperatur des in die Kammer eingeleiteten Fluidisierungsgases: etwa 100°C) die oben beschriebenen Nickeloxid-haltigen $\gamma$-$Al_2O_3$-Kugeln fluidisiert. Anschließend wurde eine wässrige Suspension kolloidaler Boehmit-Partikel (d.h. kolloidale AlO(OH)-Partikel) in die Wirbelschichtkammer eingedüst. Die eingedüste Suspension wurde durch die Düse fein zerstäubt und die Boehmit-Partikel schieden sich auf der Oberfläche der $\gamma$-$Al_2O_3$-Kugeln ab.

Nach 60 Minuten wurden beschichtete $\gamma$-$Al_2O_3$-Kugeln der Wirbelschichtkammer entnommen. Diese beschichteten Kugeln wurden anschließend für drei Stunden einer thermischen Behandlung bei 550°C unterzogen.

Die poröse Beschichtung wies eine Dicke von etwa 105 $\mu$m auf. Mittels Quecksilberporosimetrie wurden die Porosität $\Theta_{PB}$ und der mittlere Porendurchmesser $d_{PB}$ der auf den $\gamma$-$Al_2O_3$-Kugeln aufgebrachten porösen Beschichtung bestimmt. Die Porosität $\Theta_{PB}$ betrug 0,282 und der mittlere Porendurchmesser $d_{PB}$ betrug 4,4 nm.

Die Eigenschaften der in diesem Beispiel hergestellten Formkörper sind nachfolgend nochmals zusammengefasst.

Als katalytisch aktiver Bereich der Formkörper fungieren jeweils die mit Nickeloxid beladenen $\gamma$-$Al_2O_3$-Kugeln (Kugeldurchmesser: 2,5 mm). Deren Porosität $\Theta_{KB}$ und mittlerer Porendurchmesser $d_{KB}$ wiesen folgende Werte auf:

$$\Theta_{KB} = 0,687$$

$$d_{KB} = 10{,}7 \text{ nm}$$

Diese mit Nickeloxid beladenen $\gamma$-$Al_2O_3$-Kugeln wurden über eine Sprühbeschichtung in einer Wirbelschichtkammer mit einer porösen Aluminiumoxidbeschichtung versehen (Schichtdicke der porösen Beschichtung: ca. 105 $\mu$m). In dieser porösen Beschichtung liegt kein katalytisch aktives Material vor. Die Porosität $\Theta_{PB}$ und der mittlere Porendurchmesser $d_{PB}$ der porösen, katalytisch inerten Beschichtung wiesen folgende Werte auf:

$$\Theta_{PB} = 0{,}282$$

$$d_{PB} = 4{,}4 \text{ nm}$$

Für die Beziehung

$$[\Theta_{PB}^{1.5} \times d_{PB} \times (d_{KB} + 150 \text{ nm})] / [\Theta_{KB}^{1.5} \times d_{KB} \times (d_{PB} + 150 \text{ nm})]$$

ergibt sich ein Wert von 0,11.
Der mittlere Porendurchmesser $d_{PB}$ und die Porosität $\Theta_{PB}$ der aufgebrachten porösen Beschichtung wurden also so auf den mittleren Porendurchmesser $d_{KB}$ und die Porosität $\Theta_{KB}$ der vorgelegten Nickeloxid-beladenen $Al_2O_3$-Kugeln abgestimmt, dass die erfindungsgemäße Beziehung erfüllt ist.

**Eigenschaften von Katalysator-Formkörpern in einer exothermen Reaktion**

In den nachfolgend beschriebenen erfindungsgemäßen Beispielen EB1 bis EB6 und den Vergleichsbeispielen VB1 bis VB5 wird anhand computergestützter Simulationen gezeigt, dass bei einer stark exothermen Reaktion das Hot-Spot-Verhalten deutlich verbessert wird (bei weiterhin sehr hohen oder zumindest ausreichend hohen Raum-Zeit-Ausbeuten), wenn

- auf einem Ausgangsformkörper, der das katalytisch aktive Material enthält, eine katalytisch inerte poröse Beschichtung aufgebracht wird und
- unter Berücksichtigung der Porosität und des mittleren Porendurchmessers des katalytisch aktiven Kerns die Porosität und der mittlere Porendurchmesser der porösen Beschichtung so gewählt sind, dass die folgende Bedingung erfüllt ist:

$$[\Theta_{PB}^{1.5} \times d_{PB} \times (d_{KB} + 150 \text{ nm})] / [\Theta_{KB}^{1.5} \times d_{KB} \times (d_{PB} + 150 \text{ nm})] < 1$$

wobei

$\Theta_{PB}$ die Porosität der porösen Beschichtung ist;
$d_{PB}$ der mittlere Porendurchmesser, in nm, der porösen Beschichtung ist;
$\Theta_{KB}$ die Porosität des katalytisch aktiven Bereichs ist;
$d_{KB}$ der mittlere Porendurchmesser, in nm, des katalytisch aktiven Bereichs ist.

Als Beispiel für eine stark exotherme Reaktion wurde die Methanisierung (Umsetzung von $CO_2$ mit Wasserstoff zu Methan) in einem wandgekühlten Festbettrohrreaktor simuliert. In der Simulation wurden für alle Beispiele identische Reaktor- bzw. Verfahrensbedingungen zugrunde gelegt. Die Beispiele unterscheiden sich lediglich hinsichtlich der Katalysator-Formkörper.
Grundlage der Simulationen ist ein heterogenes Modell eines wandgekühlten Festbettrohrreaktors, welches den Zustand des Reaktors entlang der Rohrachse und entlang des Radius der katalytisch aktiven Formkörper (hier Kugeln) wiedergibt. Der Zustand des Reaktors ist hierbei durch die Konzentration aller chemischen Spezies und die Temperatur beschrieben. Folgende Annahmen und Gleichungen sind Grundlage des Modells:

• keine Druckgradienten im Reaktor,
• keine axiale Dispersion und Wärmeleitung im Reaktor,

- ideales Gasverhalten,
- konstante Kühlmitteltemperatur,
- konstanter Wandwärmeübergangskoeffizient auf der Außenseite des Reaktorrohres,
- Wärmetransportwiderstand der Reaktorwand wird vernachlässigt,
- Wandwärmeübergangskoeffizient auf der Rohrinnenseite wird nach der Korrelation von Martin und Nilles berechnet, siehe H. Martin and M. Nilles, Chemie Ingenieur Technik, 65(12):1468-1477, 1993,
- effektive radiale Wärmeleitfähigkeit im Festbett wird nach der Korrelation von Yagi und Kunii berechnet, siehe S. Yagi und D. Kunii, AIChE Journal, 3(3):373-381, 1957,
- effektive radiale Wärmeleitfähigkeit und Wandwärmeübergangskoeffizient auf der Rohrinnenseite werden nach Froment zusammengefasst, siehe G. F. Froment, Industrial & Engineering Chemistry, 59(2):18-27, 1967,
- Wärmeleitfähigkeit des Festbetts anhand bekannter Daten und Gleichungen berechnet,
- Stoff- und Wärmeübergangskoeffizienten an die katalytisch aktiven Formkörper werden nach N. Wakao et al., Chemical Engineering Science, 33(10):1375-1384, 1978, berechnet,
- Stofftransport in den katalytisch aktiven Formkörpern ist nach dem ersten Fick'schen Ansatz berechnet,
- Wärmetransport in den Katalysator-Formkörpern wird nach dem Fourier'schen Ansatz berechnet,
- effektive Wärmeleitfähigkeit der Katalysator-Formkörper ergibt sich aus dem Model nach Harriot, siehe P. Harriott, The Chemical Engineering Journal, 10(1):65-71, 1975.

Die Reaktionskinetik der Methanisierung von $CO_2$ wird von Koschany et al., Applied Catalysis B: Environmental, 181, 2016, S. 504-516 beschrieben. Die katalytische Aktivität der Formkörper ist in allen Beispielen in Abwesenheit von Transportwiderständen gleich. Das Modell ist in das kommerzielle Rechenprogramm Matlab implementiert und mit Hilfe des Newton-Lösers der CasADi-Toolbox gelöst worden.

Die in der Simulation verwendeten Modellparameter sind nachfolgend aufgelistet:

| | |
|---|---|
| - Rohrlänge: | 1,5 m |
| - Rohrdurchmesser: | 3 cm |
| - Durchmesser der kugelförmigen Katalysator-Formkörper: | 2,5 mm |
| - Hohlraumanteil des Festbetts: | 0,4 |
| - Wandwärmeübergangskoeffizient auf Rohraußenseite: | 500 W/(m$^2$*K) |
| - Kühlmitteltemperatur: | 500 K |

| | |
|---|---|
| - Gaseintrittsgeschwindigkeit: | 1 m/s |
| - Reaktordruck: | 5 bar |
| - Feststoffwärmeleitfähigkeit: | 2,5 W/(mK) |
| - Stoffmengenanteil Wasserstoff auf Einlassseite: | 0,8 |
| - Stoffmengenanteil Kohlenstoffdioxid auf Einlassseite: | 0,2 |
| - Temperatur auf Einlassseite: | 500 K |

Die Katalysator-Formkörper der Vergleichsbeispiele VB1-VB5 und der erfindungsgemäßen Beispiele EB1 bis EB6:

In den Vergleichsbeispielen VB1-VB3 ist das katalytisch aktive Material homogen in den Formkörpern verteilt.

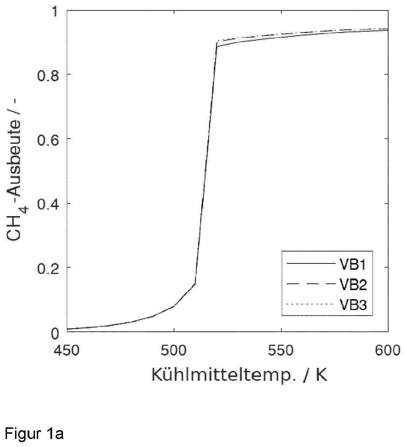

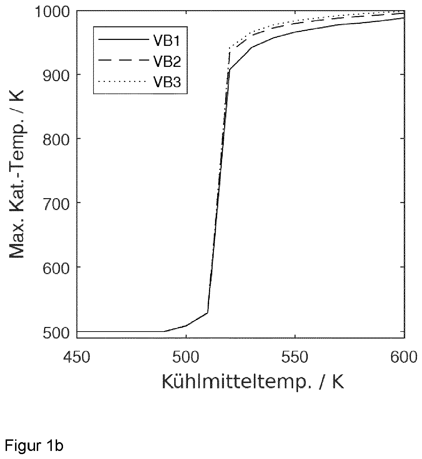

Für VB1-VB3 zeigt Figur 1a die Methan-Ausbeute als Funktion der Kühlmitteltemperatur, während Figur 1b die maximale Katalysatortemperatur als Funktion der Kühlmitteltemperatur zeigt.

In Vergleichsbeispiel VB4 und den erfindungsgemäßen Beispielen EB1 bis EB3 weist der Katalysator-Formkörper jeweils eine sog. Egg-Yolk-Struktur auf, d.h. ein katalytisch aktiver Bereich ist von einer porösen, katalytisch inerten Schale umschlossen. VB4 und EB1-EB3 weisen einen identischen katalytisch aktiven Kern auf. Weiterhin weist die den katalytisch aktiven Kern umschließende poröse Beschichtung in VB4 und EB1-EB3 jeweils den gleichen mittleren Porendurchmesser $d_{PB}$ auf. Lediglich die Porosität $\Theta_{PB}$ der porösen Beschichtung wird variiert. Während in EB1 bis EB3 die Porosität $\Theta_{PB}$ der Schale so gewählt ist, dass die Bedingung

$$[\Theta_{PB}^{1.5} \times d_{PB} \times (d_{KB} + 150 \text{ nm})] / [\Theta_{KB}^{1.5} \times d_{KB} \times (d_{PB} + 150 \text{ nm})] < 1$$

erfüllt ist, trifft dies für Vergleichsbeispiel VB4 nicht zu.

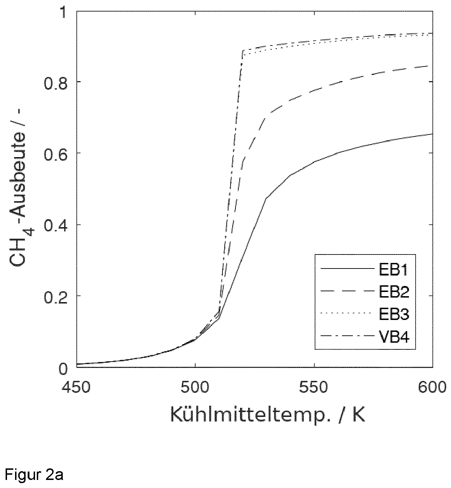

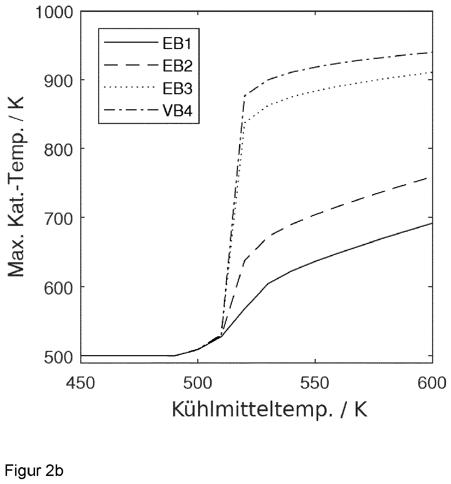

Für VB4 und EB1-EB3 zeigt Figur 2a die Methan-Ausbeute als Funktion der Kühlmitteltemperatur, während Figur 2b die maximale Katalysatortemperatur als Funktion der Kühlmitteltemperatur zeigt.

Auch in Vergleichsbeispiel VB5 und den erfindungsgemäßen Beispielen EB4 bis EB6 weist der Katalysator-Formkörper jeweils eine sog. Egg-Yolk-Struktur auf, d.h. ein katalytisch aktiver Bereich ist von einer porösen, katalytisch inerten Beschichtung umschlossen. VB5 und EB4-EB6 weisen einen identischen katalytisch aktiven Kern auf. Weiterhin weist die den katalytisch aktiven Kern umschließende poröse Beschichtung in VB5 und EB4-EB6 jeweils die gleiche Porosität $\Theta_{PB}$ auf. Lediglich der mittlere Porendurchmesser $d_{PB}$ der porösen Beschichtung wird variiert. Während in EB4 bis EB6 der mittlere Porendurchmesser $d_{PB}$ der porösen Beschichtung so gewählt ist, dass die Bedingung

$$[\Theta_{PB}^{1,5} \times d_{PB} \times (d_{KB} + 150 \text{ nm})] / [\Theta_{KB}^{1,5} \times d_{KB} \times (d_{PB} + 150 \text{ nm})] < 1$$

erfüllt ist, trifft dies für Vergleichsbeispiel VB5 nicht zu.

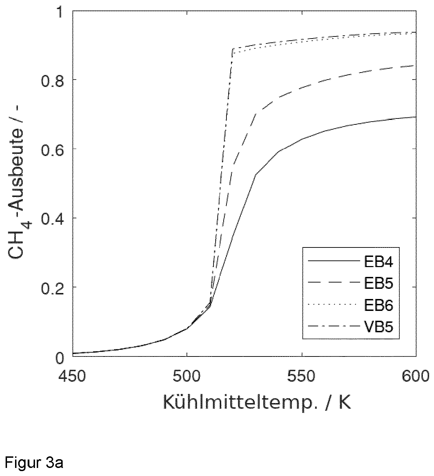

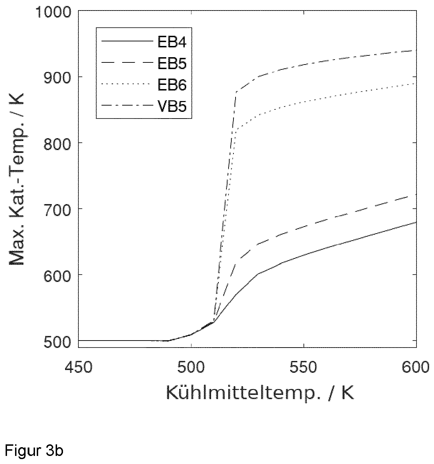

Für VB5 und EB4-EB6 zeigt Figur 3a die Methan-Ausbeute als Funktion der Kühlmitteltemperatur, während Figur 3b die maximale Katalysatortemperatur als Funktion der Kühlmitteltemperatur zeigt.

In den nachfolgenden Tabellen 1a und 1b sind die Eigenschaften der Katalysator-Formkörper zusammengefasst.

Tabelle 1a: Eigenschaften der Katalysator-Formkörper

| Beisp. | Katalytisch aktives Material | Poröse Beschichtung | | Katalytisch aktiver Kern | |
|---|---|---|---|---|---|
| | | Porosität | Mittlerer PorenDurchmesser [nm] | Porosität | Mittlerer Porendurchmesser [nm] |
| VB1 | Homogen verteilt | --- | --- | 0,15 | 1000 |
| VB2 | Homogen verteilt | --- | --- | 0,45 | 1000 |
| VB3 | Homogen verteilt | --- | --- | 0,75 | 1000 |
| VB4 | Inhomogen verteilt, Egg-Yolk | 0,45 | 1000 | 0,45 | 1000 |
| EB1 | Inhomogen verteilt, Egg-Yolk | 0,06 | 1000 | 0,45 | 1000 |
| EB2 | Inhomogen verteilt, Egg-Yolk | 0,1 | 1000 | 0,45 | 1000 |
| EB3 | Inhomogen verteilt, Egg-Yolk | 0,3 | 1000 | 0,45 | 1000 |
| VB5 | Inhomogen verteilt, Egg-Yolk | 0,45 | 1000 | 0,45 | 1000 |
| EB4 | Inhomogen verteilt, Egg-Yolk | 0,45 | 5 | 0,45 | 1000 |
| EB5 | Inhomogen verteilt, Egg-Yolk | 0,45 | 10 | 0,45 | 1000 |
| EB6 | Inhomogen verteilt, Egg-Yolk | 0,45 | 100 | 0,45 | 1000 |

Tabelle 1b: Eigenschaften der Katalysator-Formkörper

| Beisp. | $[\Theta_{PB}^{1.5} \times d_{PB} \times (d_{KB} + 150\ nm)] / [\Theta_{KB}^{1.5} \times d_{KB} \times (d_{PB} + 150\ nm)]$ |
|---|---|
| VB1 | ---- |
| VB2 | ---- |
| VB3 | ---- |
| VB4 | 1,0 |
| EB1 | 0,05 |
| EB2 | 0,1 |
| EB3 | 0,54 |
| VB5 | 1,0 |
| EB4 | 0,04 |
| EB5 | 0,07 |
| EB6 | 0,46 |

In Figur 1a ist die Ausbeute an $CH_4$ am Reaktorausgang und in Figur 1b die damit verbundene, maximale Temperatur des Katalysator-Formkörpers im Reaktor über der Kühlmitteltemperatur aufgetragen. Bei geringen Kühlmitteltemperaturen liegen auch geringe maximale Formkörpertemperaturen vor. Aufgrund der geringen Temperaturen ist jedoch auch die Ausbeute an $CH_4$ im Reaktor gering. Wegen der stark nichtlinearen Abhängigkeit der Reaktionsgeschwindigkeit von der Temperatur (Arrhenius-Gleichung) steigt ab einer Kühlmittel-Temperatur von ca. 515 K der Umsatz im Reaktor stark an und es wird eine Produktausbeute von mehr als 80 % erreicht. Aufgrund der Wärmefreisetzung der Reaktion ist hieran jedoch ein sehr starker Anstieg in der maximalen Formkörpertemperatur gekoppelt. Eine Variation der Porosität hat nur einen sehr geringen Einfluss auf die maximale Temperatur des Katalysatorformkörpers.

Aufgrund der sehr hohen maximalen Temperatur des Formkörpers ist in VB1-VB3 mit einer Schädigung oder einer signifikant verkürzten Lebensdauer des Katalysators zu rechnen.

Aus Vergleichsbeispiel VB4 in Figur 2a/b und Vergleichsbeispiel VB5 in Figur 3a/b ergibt sich im Vergleich zu den Vergleichsbeispielen VB1-VB3 in Figur 1a/b Folgendes:
Die Anwesenheit einer porösen, katalytisch inerten Beschichtung auf dem katalytisch aktiven Kern führt zu einer Absenkung der maximalen Temperatur des Formkörpers, während die Methan-Ausbeute weiterhin auf einem sehr hohen Level gehalten werden kann.

Aus den erfindungsgemäßen Beispielen EB1-EB3 und dem Vergleichsbeispiel VB4 in Figur 2a/b lässt sich Folgendes schließen:
Wird für die poröse Beschichtung die Porosität $\Theta_{PB}$ weiter reduziert, so dass

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1,0$$

erfolgt auch eine weitere Absenkung der maximalen Temperatur des Katalysator-Formkörpers.

In den erfindungsgemäßen Beispielen EB1 und EB2 findet eine signifikante Absenkung der Temperatur des Katalysator-Formkörpers statt, was wiederum zu einer drastischen Erhöhung der Katalysatorlebenszeit führt. Sowohl EB1 als auch EB2 weisen noch ausreichend hohe Methan-Ausbeuten auf. Im erfindungsgemäßen Beispiel EB3 fällt zwar die Absenkung der Temperatur des Formkörpers geringer aus als in EB1 und EB2, dafür kann die Methan-Ausbeute auf einem sehr hohen Level gehalten werden.

Aus den erfindungsgemäßen Beispielen EB4-EB6 und dem Vergleichsbeispiel VB5 in Figur 3a/b lässt sich Folgendes schließen:
Wird für die poröse Beschichtung der mittlere Porendurchmesser $d_{PB}$ weiter reduziert, so dass

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1,0$$

erfolgt auch eine weitere Absenkung der maximalen Temperatur des Katalysator-Formkörpers.

In den erfindungsgemäßen Beispielen EB4 und EB5 findet eine signifikante Absenkung der Temperatur des Katalysator-Formkörpers statt, was wiederum zu einer drastischen Erhöhung der Katalysatorlebenszeit führt. Sowohl EB4 als auch EB5 weisen noch ausreichend hohe Methan-Ausbeuten auf. In dem erfindungsgemäßen Beispiel EB6 fällt zwar die Absenkung der Temperatur des Katalysator-Formkörpers geringer aus als in EB4 und EB5, dafür kann die Methan-Ausbeute auf einem sehr hohen Level gehalten werden.

**Patentansprüche**

1. Formkörper, der ein katalytisch aktives Material enthält und der einen katalytisch aktiven Bereich sowie eine poröse Beschichtung, die auf dem katalytisch aktiven Bereich vorliegt, umfasst, wobei

   - mindestens 75 Gew% des katalytisch aktiven Materials des Formkörpers in dem katalytisch aktiven Bereich vorliegen, und
   - die poröse Beschichtung und der katalytisch aktive Bereich der folgenden Bedingung genügt:

$$[\Theta_{PB}^{1.5} \times d_{PB} \times (d_{KB} + 150\ nm)] / [\Theta_{KB}^{1.5} \times d_{KB} \times (d_{PB} + 150\ nm)] < 1,0$$

   wobei

   $\Theta_{PB}$ die Porosität der porösen Beschichtung ist;
   $d_{PB}$ der mittlere Porendurchmesser, in nm, der porösen Beschichtung ist;
   $\Theta_{KB}$ die Porosität des katalytisch aktiven Bereichs ist;
   $d_{KB}$ der mittlere Porendurchmesser, in nm, des katalytisch aktiven Bereichs ist.

2. Formkörper nach Anspruch 1, wobei der katalytisch aktive Bereich ein poröses Trägermaterial, auf dem das katalytisch aktive Material dispergiert ist, enthält.

3. Formkörper nach Anspruch 1 oder 2, wobei die Porosität $\Theta_{KB}$ des katalytisch aktiven Bereichs 0,2 bis 0,8 beträgt.

4. Formkörper nach einem der vorstehenden Ansprüche, wobei die Porositäten und mittleren Porendurchmesser des katalytisch aktiven Bereichs und der darauf vorliegenden porösen Beschichtung der folgenden Bedingung genügen:

$$0,01 < [\Theta_{PB}^{1.5} \times d_{PB} \times (d_{KB} + 150\ nm)] / [\Theta_{KB}^{1.5} \times d_{KB} \times (d_{PB} + 150\ nm)] \leq 0,75$$

5. Formkörper nach einem der vorstehenden Ansprüche, wobei die poröse Beschichtung ein Oxid, ein Nitrid, ein Carbid und/oder elementaren Kohlenstoff enthält.

6. Formkörper nach einem der vorstehenden Ansprüche, wobei der Formkörper einen volumenäquivalenten Kugeldurchmesser im Bereich von 1,0 mm bis 50 mm, bevorzugter 1,0 bis 20 mm aufweist; und/oder der Formkörper in Form eines Pellets, einer Kugel, eines Rings, einer Tablette, eines Strangs, eines Zylinders, eines schwammförmigen Formkörpers oder eines wabenförmigen Formkörpers vorliegt.

7. Formkörper nach einem der vorstehenden Ansprüche, wobei die poröse Beschichtung des Formkörpers eine Schichtdicke im Bereich von 10 $\mu$m bis 2000 $\mu$m, bevorzugter 50 $\mu$m bis 1000 $\mu$m aufweist.

8. Formkörper nach einem der vorstehenden Ansprüche, wobei der mittlere Porendurchmesser $d_{PB}$ der porösen Beschichtung $\leq$ 200 nm, bevorzugter $\leq$ 150 nm, noch bevorzugter $\leq$ 50 nm ist.

9. Formkörper nach einem der vorstehenden Ansprüche, wobei das gesamte katalytisch aktive Material des Formkörpers in dem katalytisch aktiven Bereich vorliegt.

10. Verwendung des Formkörpers nach einem der Ansprüche 1 bis 9 als Katalysator für eine exotherme Gasphasenreaktion.

11. Verwendung nach Anspruch 10, wobei die exotherme Gasphasenreaktion eine Standardreaktionsenthalpie $\Delta H° \leq$ -10 kJ/mol, bevorzugter $\leq$ -50 kJ/mol, noch bevorzugter $\leq$ -100 kJ/mol aufweist.

12. Verfahren zur exothermen Umsetzung gasförmiger Edukte, wobei die Edukte in einem Reaktor mit dem Formkörper nach einem der Ansprüche 1 bis 9 in Kontakt gebracht werden.

13. Verfahren nach Anspruch 12, wobei der Reaktor ein Festbettreaktor, ein Wirbelschichtreaktor, ein Wandreaktor, ein Membranreaktor, ein Mikroreaktor, ein Wabenreaktor oder ein Plattenreaktor ist.

14. Verfahren nach Anspruch 12 oder 13, wobei die exotherme Umsetzung gasförmiger Edukte eine Hydrierung, eine Oxidation, eine Acetylierung, eine Aminierung oder eine Nitrilierung ist.

15. Verfahren nach Anspruch 14, wobei die Hydrierung eine Methanisierung ist.

**Claims**

1. Shaped body which contains a catalytically active material and which comprises a catalytically active region and a porous coating which is present on the catalytically active region, wherein

   - at least 75% by weight of the catalytically active material of the shaped body are present in the catalytically active region and
   - the porous coating and the catalytically active region satisfy the following condition:

$$[\Theta_{PB}^{1.5} \ x \ d_{PB} \ x \ (d_{KB} + 150 \ nm)] \ / \ [\Theta_{KB}^{1.5} \ x \ d_{KB} \ x \ (d_{PB} + 150 \ nm)] < 1.0$$

   where

   $\Theta_{PB}$ is the porosity of the porous coating;
   $d_{PB}$ is the average pore diameter, in nm, of the porous coating;
   $\Theta_{KB}$ is the porosity of the catalytically active region;
   $d_{KB}$ is the average pore diameter, in nm, of the catalytically active region.

2. Shaped body according to Claim 1, wherein the catalytically active region contains a porous support material on which the catalytically active material is dispersed.

3. Shaped body according to Claim 1 or 2, wherein the porosity $\Theta_{KB}$ of the catalytically active region is from 0.2 to 0.8.

4. Shaped body according to any of the preceding claims, wherein the porosities and average pore diameters of the catalytically active region and of the porous coating present thereon satisfy the following condition:

$$0.01 < [\Theta_{PB}^{1.5} \ x \ d_{PB} \ x \ (d_{KB} + 150 \ nm)] \ / \ [\Theta_{KB}^{1.5} \ x \ d_{KB} \ x \ (d_{PB} + 150 \ nm)] \leq 0.75.$$

5. Shaped body according to any of the preceding claims, wherein the porous coating contains an oxide, a nitride, a carbide and/or elemental carbon.

6. Shaped body according to any of the preceding claims, wherein the shaped body has a volume-equivalent sphere diameter in the range from 1.0 mm to 50 mm, more preferably from 1.0 to 20 mm; and/or the shaped body is present in the form of a pellet, a sphere, a ring, a tablet, an extrudate, a cylinder, a sponge-like shaped body or a honeycomb shaped body.

7. Shaped body according to any of the preceding claims, wherein the porous coating of the shaped body has a layer thickness in the range from 10 $\mu$m to 2000 pm, more preferably from 50 $\mu$m to 1000 $\mu$m.

8. Shaped body according to any of the preceding claims, wherein the average pore diameter $d_{PB}$ of the porous coating is $\leq 200$ nm, more preferably $\leq 150$ nm, even more preferably $\leq 50$ nm.

**9.** Shaped body according to any of the preceding claims, wherein the entire catalytically active material of the shaped body is present in the catalytically active region.

**10.** Use of the shaped body according to any of Claims 1 to 9 as catalyst for an exothermic gas-phase reaction.

**11.** Use according to Claim 10, wherein the exothermic gas-phase reaction has a standard enthalpy of reaction $\Delta H°$ of $\leq$ -10 kJ/mol, more preferably $\leq$ -50 kJ/mol, even more preferably $\leq$ -100 kJ/mol.

**12.** Process for the exothermic reaction of gaseous starting materials, wherein the starting materials are brought into contact with the shaped body according to any of Claims 1 to 9 in a reactor.

**13.** Process according to Claim 12, wherein the reactor is a fixed-bed reactor, a fluidized-bed reactor, a wall reactor, a membrane reactor, a microreactor, a honeycomb reactor or a plate reactor.

**14.** Process according to Claim 12 or 13, wherein the exothermic reaction of gaseous starting materials is a hydrogenation, an oxidation, an acetylation, an amination or a nitrilation.

**15.** Process according to Claim 14, wherein the hydrogenation is a methanation.


**Revendications**

**1.** Corps moulé qui contient un matériau catalytiquement actif et qui comprend une zone catalytiquement active ainsi qu'un revêtement poreux qui se trouve sur la zone catalytiquement active,

- au moins 75 % en poids du matériau catalytiquement actif du corps moulé se trouvant dans la zone catalytiquement active, et
- le revêtement poreux et la zone catalytiquement active satisfaisant à la condition suivante :

$$[\Theta_{PB}^{1,5} \times d_{PB} \times (d_{KB} + 150 \text{ nm})] / [\Theta_{KB}^{1,5} \times d_{KB} \times (d_{PB} + 150 \text{ nm})] < 1,0,$$

$\Theta_{PB}$ étant la porosité du revêtement poreux ;
$d_{PB}$ étant le diamètre moyen de pores, en nm, du revêtement poreux ;
$\Theta_{KB}$ étant la porosité de la zone catalytiquement active ;
$d_{KB}$ étant le diamètre moyen de pores, en nm, de la zone catalytiquement active.

**2.** Corps moulé selon la revendication 1, la zone catalytiquement active contenant un matériau de support poreux sur lequel le matériau catalytiquement actif est dispersé.

**3.** Corps moulé selon la revendication 1 ou 2, la porosité $\Theta_{KB}$ de la zone catalytiquement active étant de 0,2 à 0,8.

**4.** Corps moulé selon l'une quelconque des revendications précédentes, les porosités et les diamètres moyens de pores de la zone catalytiquement active et du revêtement poreux se trouvant sur celle-ci satisfaisant la condition suivante :

$$0,01 < [\Theta_{PB}^{1,5} \times d_{PB} \times (d_{KB} + 150 \text{ nm})] / [\Theta_{KB}^{1,5} \times d_{KB} \times (d_{PB} + 150 \text{ nm})] \leq 0,75.$$

**5.** Corps moulé selon l'une quelconque des revendications précédentes, le revêtement poreux contenant un oxyde, un nitrure, un carbure et/ou du carbone élémentaire.

**6.** Corps moulé selon l'une quelconque des revendications précédentes, le corps moulé présentant un diamètre sphérique équivalent en volume dans la plage de 1,0 mm à 50 mm, plus préférablement de 1,0 à 20 mm et/ou le corps moulé se trouvant sous forme d'une pastille, d'une bille, d'un anneau, d'un comprimé, d'un brin, d'un cylindre, d'un corps moulé sous forme d'éponge ou d'un corps moulé sous forme de nid d'abeilles.

**7.** Corps moulé selon l'une quelconque des revendications précédentes, le revêtement poreux du corps moulé présentant une épaisseur de couche dans la plage de 10 $\mu$m à 2 000 pm, plus préférablement de 50 $\mu$m à 1 000 $\mu$m.

**8.** Corps moulé selon l'une quelconque des revendications précédentes, le diamètre moyen de pores $d_{PB}$ du revêtement poreux étant $\leq$ 200 nm, plus préférablement $\leq$ 150 nm, encore plus préférablement $\leq$ 50 nm.

**9.** Corps moulé selon l'une quelconque des revendications précédentes, le matériau catalytiquement actif total du corps moulé se trouvant dans la zone catalytiquement active.

**10.** Utilisation du corps moulé selon l'une quelconque des revendications 1 à 9 en tant que catalyseur pour une réaction exothermique en phase gazeuse.

**11.** Utilisation selon la revendication 10, la réaction exothermique en phase gazeuse présentant une enthalpie standard de réaction $\Delta H° \leq$ -10 kJ/mole, plus préférablement $\leq$ -50 kJ/mole, encore plus préférablement $\leq$ -100 kJ/mole.

**12.** Procédé de transformation exothermique d'éduits sous forme gazeuse, les éduits étant mis en contact dans un réacteur avec le corps moulé selon l'une quelconque des revendications 1 à 9.

**13.** Procédé selon la revendication 12, le réacteur étant un réacteur à lit fixe, un réacteur à couche tourbillonnante, un réacteur à paroi, un réacteur à membrane, un microréacteur, un réacteur en nid d'abeilles ou un réacteur à plaques.

**14.** Procédé selon la revendication 12 ou 13, la transformation exothermique d'éduits sous forme de gaz étant une hydrogénation, une oxydation, une acétylation, une amination ou une nitrilation.

**15.** Procédé selon la revendication 14, l'hydrogénation étant une méthanation.

Figur 1a

Figur 1b

Figur 2a

Figur 2b

Figur 3a

Figur 3b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017161953 A1
- DE 2813329 A1
- US 2012302811 A1
- US 6211255 B1

**In der Beschreibung aufgeführte Nicht-Patentliteratur**

- Industrial Catalysis - A Practical Approach. **JENS HAGEN.** Catalyst Shapes and Production of Heterogeneous Catalysts. Wiley-VCH Verlag, 2006
- Heterogeneous Catalysis and Solid Catalysts. **H. KNÖZINGER et al.** Ullmann's Encyclopedia of Industrial Chemistry. Wiley-VCH Verlag, 2009
- **S. RÖNSCH et al.** *Fuel,* 2016, vol. 166, 276-296
- **S. HWANG et al.** *Chem. Eng. Comm,* 2009, vol. 196, 616-642
- **L. KERSHENBAUM et al.** *Chemical Engineering Science,* 2001, vol. 56, 651-658
- Catalyst Forming. **F. SCHÜTH ; M. HESSE.** Handbook of Heterogeneous Catalysis. Wiley-VCH, 2008, 676-699
- Sol-Gel Process. **M.V. LANDAU.** Handbook of heterogeneous catalysis. Wiley-VCH Verlag, 2008, vol. 1, 119-160
- **C.J. BRINKER et al.** *Journal of Membrane Science,* 1994, vol. 94, 85-102
- General Principles for the Synthesis and Modification of Porous Materials. **F. SCHÜTH.** Handbook of Porous Solids. Wiley-VCH, 2002, vol. 1, 622-629
- **H. MARTIN ; M. NILLES.** *Chemie Ingenieur Technik,* 1993, vol. 65 (12), 1468-1477
- **S. YAGI ; D. KUNII.** *AIChE Journal,* 1957, vol. 3 (3), 373-381
- **G. F. FROMENT.** *Industrial & Engineering Chemistry,* 1967, vol. 59 (2), 18-27
- **N. WAKAO et al.** *Chemical Engineering Science,* 1978, vol. 33 (10), 1375-1384
- **P. HARRIOTT.** *The Chemical Engineering Journal,* 1975, vol. 10 (1), 65-71
- **KOSCHANY et al.** *Applied Catalysis B: Environmental,* 2016, vol. 181, 504-516